(19) **Europäisches Patentamt**

**European Patent Office**

**Office européen des brevets**

(11) **EP 0 943 297 B1**

(12) **EUROPÄISCHE PATENTSCHRIFT**

(45) Veröffentlichungstag und Bekanntmachung des Hinweises auf die Patenterteilung:
**08.03.2000 Patentblatt 2000/10**

(51) Int Cl.⁷: $A61F\ 2/00$, $A61F\ 2/36$

(21) Anmeldenummer: **98102320.3**

(22) Anmeldetag: **11.02.1998**

(54) **Femorale Hüftgelenkprothese**

Femoral part for a hip joint prosthesis

Partie fémoral d'une prothese d'articulation de hanche

(84) Benannte Vertragsstaaten:
**AT DE ES GB**

(43) Veröffentlichungstag der Anmeldung:
**22.09.1999 Patentblatt 1999/38**

(73) Patentinhaber: **PLUS Endoprothetik AG**
**6343 Rotkreuz (CH)**

(72) Erfinder: **Schmotzer, Hans F.**
**5742 Kölliken (CH)**

(74) Vertreter: **Patentanwälte**
**Schaad, Balass, Menzl & Partner AG**
**Dufourstrasse 101**
**Postfach**
**8034 Zürich (CH)**

(56) Entgegenhaltungen:
**EP-A- 0 790 045         WO-A-91/18561**
**US-A- 5 156 627**

## Beschreibung

Die Erfindung betrifft eine femorale Hüftgelenkprothese, zum Implantieren mittels Zement gemäss dem Oberbegriff von Anspruch 1.

Die Offenlegungsschrift WO 91/18561 A1 offenbart eine femorale Hüftgelenkprothese, welche als ein keilförmiger, intramedullärer Schaft mit kragenloser Schulter ausgestaltet ist. Um einen derartigen Schaft im Kanal eines Femurs festzuhalten wird eine Art von Knochenzement verwendet, welche üblicherweise eine Mischung von Polymethylmethacrylat (hier nachfolgend PMMA)-Polymer und Methylmethacrylatmonomer umfasst, und fakultativ ein Styrol-Copolymer von PMMA enthält. Diese und andere Arten von Zement, die zum Zweck verwendet werden, den Schaft im Kanal des Femurknochens festzuhalten, unterliegen einer als Kriechen bekannten Erscheinung. Obwohl der Knochenzement im ausgehärteten Zustand starr zu sein scheint, unterliegt er mit der Zeit winzigen Bewegungen, was zu einer Störung der Mikroverzahnung der Zement/Implantat-Grenzfläche sowie der Zement/Knochen-Grenzfläche führt. Dieser Effekt kann mit der Zeit ein Lockern des Schaftes verursachen. Der bekannte Schaft ist derart ausgestaltet, dass zwischen dem Knochenzement und der Oberfläche des Schaftes eine geringe Adhäsion besteht. Tritt ein Kriechen von Knochenzement auf, so sinkt der keilförmige Schaft im Knochenzement leicht ein und setzt sich automatisch wieder innerhalb des Knochenzementes fest. Um dabei ein Lockern des Schaftes zu verhindern ist es von entscheidender Bedeutung, dass das Kriechen des Zementes und das Einsinken des Schaftes möglichst zu keiner Störung der Mikroverzahnung der Zement/Knochen-Grenzfläche führt.

Nachteilig am bekannten Schaft ist die Tatsache, dass durch das Einsinken des Schaftes im Femur insbesondere an der Zement/Implantat-Grenzfläche, jedoch auch an der Zement/Knochen-Grenzfläche, die auf den Knochenzement ausgeübte Druckkraft zunimmt, wobei insbesondere an Stellen mit hoher Druckkraftzunahme ein übermässiges Kriechen oder eine Rissbildung des Knochenzementes auftritt.

Es ist Aufgabe der vorliegenden Erfindung eine femorale Hüftgelenkprothese zum Implantieren mittels Zement derart weiterzubilden, dass der Knochenzement einer geringeren Belastung ausgesetzt ist, und insbesondere eine geringe Störung der Mikroverzahnung an der Zement/Knochen-Grenzfläche auftritt.

Diese Aufgabe wird gelöst mit einer femoralen Hüftgelenkprothese aufweisend die Merkmale von Anspruch 1. Die Unteransprüche 2 bis 9 beziehen sich auf weitere, vorteilhafte Ausgestaltungen der Erfindung.

Die Erfindung wird insbesondere gelöst mit einer femoralen Hüftgelenkprothese zum Implantieren mittels Zement, aufweisend eine kragenlose Schulter am proximalen Ende und einen geradlinig von der Schulter zum distalen Ende verlaufenden Schaft, welcher sich vom proximalen zum distalen Ende hin verjüngt, wobei der Schaft einen viereckförmig ausgestalteten Querschnitt sowie eine anteriore Seitenfläche und eine posteriore Seitenfläche aufweist, und die beiden Seitenflächen in Verlaufsrichtung des Schaftes in einem ersten, an der Schulter beginnenden Teilabschnitt eine logarithmisch verlaufende Krümmung aufweisen, und die beiden Seitenflächen anschliessend in einem zweiten Teilabschnitt geradlinig und konvergierend verlaufen.

Die erfindungsgemässe femorale Hüftgelenkprothese, im weiteren auch als Femurschaft bezeichnet, weist eine anteriore und eine posteriore Seitenfläche auf, welche in Längsrichtung in je einem ersten, an der Schulter beginnenden Teilabschnitt eine logarithmisch verlaufende Krümmung aufweisen, derart, dass sich der Querschnitt des Schaftes zum distalen Ende hin verjüngt. Die anteriore und posteriore Seitenfläche weisen, anschliessend an den ersten Teilabschnitt, je einen zweiten Teilabschnitt auf, welche zum distalen Ende hin geradlinig und gegenseitig konvergierend verlaufen.

Der erfindungsgemässe Femurschaft weist den Vorteil auf, dass dessen Formgebung in Längsrichtung etwa dem anatomischen Verlauf des Markkanals des Femurs entspricht, welcher in einem ersten, oberen Teilabschnitt eine trompetenförmige Gestalt aufweist, wogegen der Markkanal in einem zweiten, unteren Teilabschnitt geradlinig verlaufende, sich in distaler Richtung leicht verengende Seitenwände aufweist. In einer bevorzugten Ausführungsform weist der Femurschaft eine derartig dem anatomischen Verlauf des Markkanals angepasste Formgebung auf, dass der zwischen dem Schaft und dem Femur zu liegen kommende Knochenzement eine etwa konstante Schichtdicke aufweist. Der erfindungsgemässe Femurschaft weist durch die logarithmisch verlaufende Krümmung zudem eine derartige Formgebung auf, dass der Zement beim Einsinken des Femurschaftes insbesondere im ersten, oberen Teilabschnitt eine möglichst gleichmässige Kompression erfährt, was im ersten Teilabschnitt eine senkrecht zur Verlaufsrichtung des Femurschaftes ausgerichtete, etwa gleichmässige Krafterhöhung bewirkt, so dass Spannungsspitzen beziehungsweise Rissbildungen im Knochenzement vermieden werden.

Der Vorteil dieser logarithmischen verlaufenden Formgebung kann natürlich auch durch eine im ersten Teilabschnitt entsprechend logarithmisch gekrümmt verlaufende laterale und mediale Seite der femoralen Hüftgelenkprothese genutzt werden.

Ein weiterer Vorteil der erfindungsgemässen Ausgestaltung der femoralen Hüftgelenkprothese ist darin zu sehen, dass durch die Vermeidung von Spannungsspitzen im Knochenzement die Zement/Knochen-Grenzfläche eine eher gleichmässige verteilte Druckbelastung aufweist. Es ist bekannt, dass an einer Zement/Knochen-Grenzfläche mit ungleichmässiger Druckbelastung an Stellen hoher Druckbelastung eine Rückbildung des Knochens auftreten kann, was ein zusätzliches Lockern des Femurschaftes zur Folge haben könnte.

Der erfindungsgemässe Femurschaft weist einen viereckförmigen Querschnitt mit Kanten auf, wobei der Querschnitt insbesondere quadratisch, rechteckig oder trapezförmig ausgestaltet ist. Es kann sich als vorteilhaft erweisen diese Kanten abzurunden, um die Kerbwirkung, welche diese Kanten beim Senken des Femurschaftes auf den Knochenzement bewirken, zu verringern. Nebst der Kerbwirkung werden natürlich auch die auf den Knochenzement wirkenden Spannungsspitzen reduziert. In einer vorteilhaften Ausgestaltung weisen die Kanten einen Radius zwischen 1 und 3 mm auf. Die erfindungsgemässe Hüftgelenkprothese weist den weiteren Vorteil auf, dass auf Grund des viereckförmigen Schaftquerschnittes am Femurschaft angreifende Torsionskräfte beziehungsweise Scherkräfte vorteilhaft auf den Knochenzement übertragbar sind. Die viereckförmige Ausgestaltung des Schaftquerschnittes sowie das Abrunden der Kanten erlaubt ein optimales Verhalten bezüglich der beiden folgenden, gegensätzlichen Anforderungen:

- Ein sehr kantig ausgestalteter Femurschaft führt beim Absinken zu hohen Spannungsspitzen oder sogar zu Spannungsrissen im Zement, so dass ein runder Querschnitt optimal wäre.
- Andererseits würde ein runder Querschnitt bei am Femurschaft angreifenden Torsionskräften eine hohe Schubspannung im Knochenzement erzeugen, so dass ein eckiger Querschnitt vorteilhafter wäre.

In einer bevorzugten Ausführungsform weist der Femurschaft eine hochpolierte Oberfläche auf, wodurch die Haftung des Zementes an der Zement/Implantat-Grenzfläche vermindert bezeihungsweise vermieden wird. Die Wirkung der Scherkräfte, die beim Absinken des Femurschaftes entlang des Schaftes auftreten oder auch die bei Torsion oder Biegung des Schaftes auftretenden Scherkräfte im Zement werden dadurch zusätzlich erheblich verringert.

Anhand der Figuren 1 bis 3 wird im folgenden ein Ausführungsbeispiel der erfindungsgemässen femoralen Hüftgelenkprothese erläutert. Es zeigen schematisch:

Fig. 1       eine femorale Hüftgelenkprothese aus medialer Richtung mit angedeutetem Femur;

Fig. 2a,2b       einen Querschnitt des Schaftes entlang der Linie A-A sowie der Linie B-B gemäss Fig. 1 bezw. Fig. 3 mit angedeutetem Markkanal und Compacta;

Fig. 3       die femorale Hüftgelenkprothese aus anteriorer Richtung mit angedeutetem Femur.

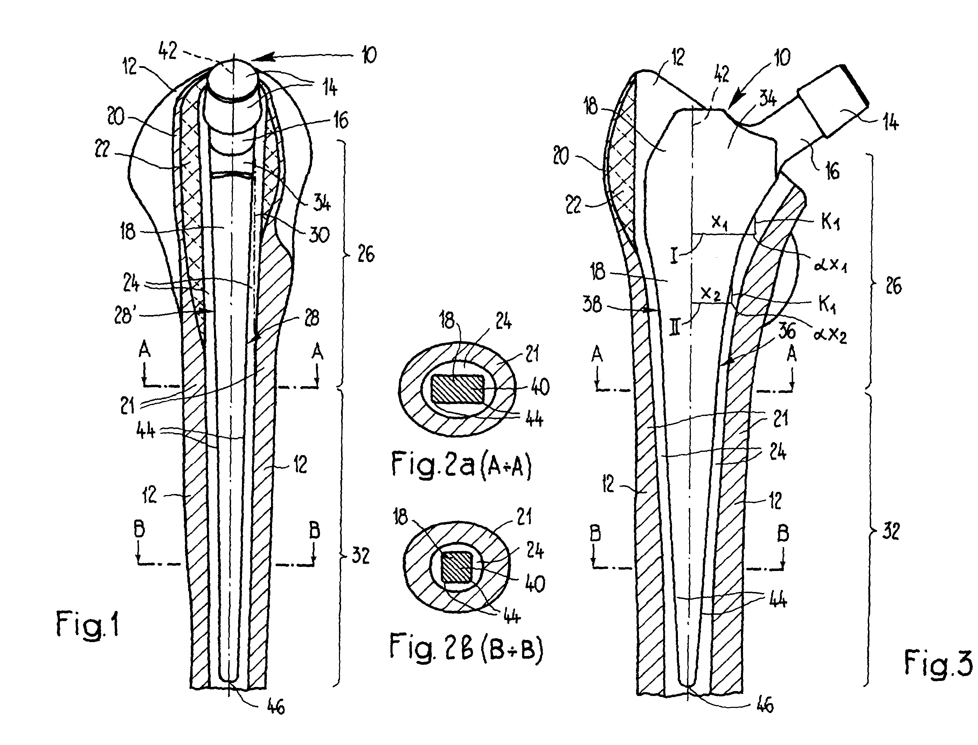

Fig. 1 zeigt eine Ansicht einer femoralen Hüftgelenkprothese 10 eingebettet in einen schematisch im Schnitt dargestellten Femur 12 aus medialer Richtung. Die femorale Hüftgelenkprothese 10 umfasst eine Schulter 34, welche auf der einen Seite nahtlos in einen Schaft 18 übergeht, und auf der anderen Seite einen Hals 16 aufweist, welcher in einen konischen Zapfen 14 mündet, auf welchem ein nicht dargestellter Gelenkkopf befestigbar ist. Am geschnitten dargestellten Femur 12 ist die Kortikalis 20, welche in die Compacta 21 übergeht, die Spongiosa 22 sowie der Markkanal 24 ersichtlich. Die femorale Hüftgelenkprothese 10 ist durch einen den dargestellen Leerraum des Markkanals 24 ausfüllenden Knochenzement im Femur 12 gehalten, wobei der einfacheren Übersichtlichkeit halber der Knochenzement nicht dargestellt ist. Der Schaft 18 weist in dessen Längsrichtung in einem ersten, an der Schulter 34 beginnenden Teilabschnitt 26 eine anteriore und eine posteriore Seitenfläche 28, 28' auf, welche eine in Längsrichtung logarithmisch laufende Krümmung aufweisen. Zur Verdeutlichung der gekrümmt verlaufenden Seitenflächen 28,28' ist beginnend an der Schulter 34 eine gestrichelt gezeichnete Vertikale 30 neben dem Schaft 18 verlaufend eingezeichnet. In einem zweiten, an den ersten Teilabschnitt 26 anschliessenden Teilabschnitt 32, weisen die anteriore und die posteriore Seitenfläche 28, 28' einen geradlinigen Verlauf auf, wobei die beiden Seitenflächen 28,28' gegen das distale Ende hin konvergierend verlaufen.

Fig. 3 zeigt dieselbe femorale Hüftgelenkprothese 10 gemäss Fig. 1 aus anteriorer Richtung. Die am proximalen Ende des Schaftes 18 befindliche Schulter 34 mit Hals 16 und Zapfen 14 ist aus dieser Perspektive gut zu erkennen. Im dargestellten Ausführungsbeispiel weist die mediale Seitenfläche 36 sowie die laterale Seitenfläche 38, wie bereits die anteriore und die posteriore Seitenfläche 28, 28', einen ersten, im proximalen Bereich angeordneten Teilabschnitt 26 auf mit einer in Längsrichtung logarithmisch gekrümmt verlaufenden Seitenfläche 36, 38, welche anschliessend in einen zweiten Teilabschnitt 32 übergehen, welche geradlinig zulaufende Flächen aufweist. Die logarithmische Krümmung der medialen und lateralen Seitenflächen 36 und 38 beginnt anschliessend an die Schulter 34.

Diese erfindungsgemässe Formgebung der Seitenflächen 28,28' und gegebenenfalls auch der Seitenflächen 36,38 ist einerseits an die natürliche, im ersten Teilabschnitt 26 trompetenförmige Gestalt und im zweiten Teilabschnitt geradlinig verlaufende Gestalt des Markraumes 24 des Femur 12 angepasst ausgestaltet. Der Schaft 18 wird vorzugsweise derart in den Markraum 24 eingesetzt, dass sich zwischen dem Schaft 18 und dem Femur eine Zementschicht mit einer vorzugsweise etwa konstanten Dicke von beispielsweise 2 mm ergibt. Die Funktion und Wirkung der logarithmischen Krümmung der Seitenflächen 28,28' und gegebenenfalls der Seitenflächen 36,38 im ersten Teilabschnitt 26 wird mit den folgenden mathematischen Ausführungen ausführlich erläutert. Wie bereits erwähnt, ist der erfindungsgemässe Schaft derart ausgelegt, dass nach erfolgtem Implantieren ein weiteres Absinken in den zementgefüllten Markkanal möglich ist. Wie in Fig. 3 dargestellt, führt die logarithmische Krümmung der Seitenflächen 28, 28', 36, 38 des Schaftes 18 dazu, dass die Änderung des Raumes, welcher der Schaft 18 beim Absinken in den zementgefüllten Markkanal 24 zusätzlich beansprucht, in Verlaufsrichtung des Schaftverlaufes konstant oder ungefähr konstant bleibt, so dass der zusätzlich etwa senkrecht auf die Zement/Knochengrenzfläche des Femurs 12 wirkende Druck in Längsrichtung gleichmässig verteilt ist, und sich dadurch an der Zement/Knochen-Grenzfläche keine Stelle mit übermässig hohem Druckwert ergibt.

Der Effekt eines leicht in den Markkanal 24 einsinkenden Schaftes 18 wird nachfolgend am Beispiel der Seitenfläche 36 erläutert, wobei diese Aussagen natürlich auch für die Seitenflächen 28, 28' und 36 zutreffen, falls sie logarithmisch gekrümmt verlaufend ausgestaltet sind. Die durch den Schaft 18 in der ursprünglichen Lage auf einer Horizontalen I von einer Mittellinie 42 des Schaftes 18 bis z.B. zur medialen Seitenfläche 36 beanspruchte Strecke ist gegeben durch $x_1$. Die durch den Schaft 18 in der ursprünglichen Lage auf einer Horizontalen II beanspruchte Strecke ist gegeben durch $x_2$. Sinkt der Schaft 18 nun um einen konstanten Verschiebungsweg $K_1$ im Femur 12 ein, so ändern sich die durch den Schaft 18 beanspruchten Strecken auf den Horizontalen I und II um $dx_1$ bzw. $dx_2$. Ein erfindungsgemässer Gedanke des dargestellten Schaftes ist, dass sich auf jeder Horizontalen i das Verhältnis der Änderung der beanspruchten Strecken $dx_i$ zu der ursprünglich durch den Schaft 18 beanspruchten Strecke $x_i$ nicht ändert, so dass das Verhältnis dieser beiden Strecken zueinander konstant, beziehungsweise gleich einer Konstanten $K_2$ ist:

$$\varepsilon(x_i) = \frac{dx_i}{x_i} = K_2 \qquad \text{Gleichung 1}$$

Das Einsinken des Schaftes 18 bewirkt somit an jeder Horizontalen i eine konstante Dehnung ε, was senkrecht zur Verlaufsrichtung des Schaftes eine konstante Dehnung des Knochenzementes und dadurch eine konstante Druckkrafterhöhung im Knochenzement bewirkt.

Damit ergibt sich auf jeder Horizontalen i die nach dem Absinken des Schaftes 18 zusätzlich beanspruchte Strecke $dx_i$ als:

$$dx_i = x_i \cdot K_2 \qquad \text{Gleichung 2}$$

Die Steigung y' der die Krümmung der Seitenfläche 36 beschreibenden Gleichung y=f($x_i$) an der Stelle der Horizontalen i ist bestimmt durch die Steigung der Tangente an dieser Stelle, welche sich wie folgt berechnet aus dem Quotient des konstanten Verschiebungsweges $K_1$ und der zusätzlich beanspruchten Strecke $dx_i$:

$$y'(x_i) = \tan(dx_i) = \frac{K_1}{dx_i} \qquad \text{Gleichung 3}$$

mit Gleichung 2 ergibt sich:

$$y'(x_i) = \frac{K_1}{K_2} \cdot \frac{1}{x_i} \qquad \text{Gleichung 4}$$

Daraus ergibt sich die folgende, allgemein Kurvengleichung für den Verlauf der Seitenflächen 28, 28' 36, 38, wobei die y-Richtung parallel zur Mittellinie 42 verläuft:

$$y(x) = K \int \frac{1}{x} dx = ln|x| + K \qquad \text{Gleichung 5}$$

$y(x)=K \int 1 \ x \ dx = 1n|x|+K$ Eine in Funktion des Logarithmus verlaufende Seitenfläche 28, 28' 36, 38 weist somit die Eigenschaft auf, dass der durch das Absinken vom Schaft 18 im zementgefüllten Markkanal 24 zusätzlich beanspruchte Raum eine konstante Dehnung ε des Knochenzementes bewirkt, was eine in Verlaufsrichtung des Schaftes 18 etwa gleichmässige vom Schaftes 18 über den Zement auf den Knochen übertragene Erhöhung des Druckes, bezw. eine gleichmässige Druckverteilung zur Folge hat.

Fig. 2a zeigt einen Querschnitt des Schaftes 18 entlang der Schnittlinie A-A, welche zudem den Übergang zwischen dem ersten und dem zweiten Teilabschnitt 26,32 bildet. Der Schaft 18 ist in dem mit Zement gefülltem Markkanal 24 eingebettet, wobei der Zement an der Zement/Knochen-Grenzfläche von der Compacta 21 umgeben ist. Der Schaft 18 weist bei der dargestellten Schnittlinie A-A einen rechteckig Querschnitt 40 auf. Gegen das distale Ende des Schaftes 18 verändert sich der Querschnitt 40 des Schaftes 18 dahingehend, dass, wie aus Fig. 2b in einem Schnitt entlang der Schnittlinie B-B ersichtlich, der Querschnitt 40 eine quadratische Form aufweist. Der Schaft 18 ist wiederum im zementgefüllten Markkanal 24 eingebettet und von der Compacta 21 umgeben. Beide dargestellten Querschnitte des Schaftes 18 weisen Kanten mit abgerundeten Ecken 44 auf. Der Schaft 18 weist beispielsweise über seine gesamte, in den Fig. 1 und 3 dargestellten Länge abgerundete Kanten 44 auf, deren Radius vorzugsweise zwischen 1 und 3mm beträgt.

Der Querschnitt des Schaftes 18 könnte auch in einer anderen viereckförmigen Form ausgestaltet sein, beispielsweise auch als ein Trapez oder ein Parallelogramm. Entweder erstreckt sich dieselbe Querschnittform über die gesamte Länge des Schaftes 18, oder die unterschiedlichen Querschnittformen gehen ineinander über. Auch ein Schaft 18 mit derart viereckförmig ausgestaltetem Querschnitt weist vorzugsweise abgerundete Kanten 44 auf, wobei die Kanten 44 des Schaftes 18 bzw. die Ecken 44 des Querschnittes 40 vorzugsweise einen Radius zwischen 1 und 3 mm aufweisen. Der Schaft 18 kann am distalen Ende 46 (Fig. 1 und 3) auch einen kreisförmigen Querschnitt aufweisen, da an dieser Stelle üblicherweise keine Übertragung von Torsions- bzw. Scherkräften erfolgt.

Ausgehend vom distalen Ende 46 können die Seitenflächen 28, 28' und 36, 38 des Schaftes 18 über eine Länge von bis zu 5mm parallel zueinander verlaufen. Dieser Endabschnitt kann zur Aufnahme einer Zentrierhülse dienen, welche das distale Ende 46 im Markkanal 24 zu zentrieren erlaubt.

Die Übergangsstelle der logarithmisch gekrümmten verlaufenden Seitenflächen 28, 28'und 36, 38 des ersten Teilabschnittes 26 zu den geradlinig konvergierenden Seitenflächen 28, 28'und 36, 38 des zweiten Teilabschnittes 32 ist im dargestellten Ausführungsbeispiel auf der Höhe der Schnittlinie A-A angeordnet. Diese Übergangsstelle kann sich beispielsweise bezüglich der Längsrichtung auch im mittleren Bereich des Schaftes 18, vorzugsweise jedoch etwa im unteren Drittel des Schaftes 18 befinden, so dass der zweite Teilabschnitt 32 sich, ausgehend vom distalen Ende 46, über die Hälfte, vorzugsweise über einen Drittel des Schaftes 18 erstreckt.

Der Femurschaft 10 besteht beispielsweise aus einem biokompatiblen metallischen Werkstoff wie Titan, einer Titanlegierung oder eine CoCrMo-Legierung. Die Hochglanz polierten Flächen sind zum Beispiel poliert mit 1µm, ½ µm, ¼ µm.

**Patentansprüche**

1. Femorale Hüftgelenkprothese (10) zum Implantieren mittels Zement aufweisend eine kragenlose Schulter (34) am proximalen Ende und einen geradlinig von der Schulter (34) zum distalen Ende (46) verlaufenden Schaft (18), der sich vom proximalen zum distalen Ende (46) hin verjüngt, dadurch gekennzeichnet, dass der Schaft (18) einen viereckförmig ausgestalteten Querschnitt (40) sowie eine anteriore Seitenfläche (28) und eine posteriore Seitenfläche (28') aufweist, dass die beiden Seitenflächen (28, 28') in Verlaufsrichtung des Schaftes (18) in einem ersten, an der Schulter (34) beginnenden Teilabschnitt (26) eine logarithmisch verlaufende Krümmung aufweisen, und dass die beiden Seitenflächen (28, 28') anschliessend in einem zweiten Teilabschnitt (32) geradlinig und konvergierend verlaufen.

2. Femorale Hüftgelenkprothese (10) nach Anspruch 1, dadurch gekennzeichnet, dass der Schaft (18) eine mediale und eine lateralen Seitenfläche (36, 38) aufweist, wobei die Seitenflächen (36, 38) jeweils in Verlaufsrichtung des Schaftes (18) in einem ersten, an der Schulter beginnenden Teilabschnitt (26) eine logarithmisch verlaufende Krümmung aufweisen, und anschliessend in einem zweiten Teilabschnitt (32) geradlinig und konvergierend verlaufen.

3. Femorale Hüftgelenkprothese (10) nach einem der vorhergehenden Ansprüche, dadurch gekennzeichnet, dass sich der zweite Teilabschnitt (32), ausgehend vom distalen Ende (46), über die Hälfte, vorzugsweise über einen Drittel des Schaftes (18) erstreckt.

4. Femorale Hüftgelenkprothese (10) nach einem der vorhergehenden Ansprüche, dadurch gekennzeichnet, dass der viereckförmige Querschnitt (40) Kanten (44) ausbildet, welche abgerundet sind.

5. Femorale Hüftgelenkprothese (10) nach Anspruch 4, dadurch gekennzeichnet, dass die abgerundeten Kanten (44) einen Radius zwischen 1 und 3 mm aufweisen.

6. Femorale Hüftgelenkprothese (10) nach einem der vorhergehenden Ansprüche, dadurch gekennzeichnet, dass der viereckförmige Querschnitt (40) trapetzförmig, rechteckig oder quadratisch ist, wobei der Querschnitt (40) vom proximalen zum distalen Ende (46) des Schaftes (18) von einer in eine andere dieser Formen übergehen kann.

7. Femorale Hüftgelenkprothese (10) nach einem der vorhergehenden Ansprüche, dadurch gekennzeichnet, dass der Schaft (18) einen dritten, distalen Endabschnitt mit einem kreisförmig ausgestalteten Querschnitt (40) aufweist.

8. Femorale Hüftgelenkprothese (10) nach einem der vorhergehenden Ansprüche, dadurch gekennzeichnet, dass die Seitenflächen (28, 28' und 36, 38) ausgehend vom distalen Ende (46) über eine Länge von bis zu 5mm parallel zueinander verlaufen.

9. Femorale Hüftgelenkprothese (10) nach einem der vorhergehenden Ansprüche, dadurch gekennzeichnet, dass zumindest die Seitenflächen (28, 28', 36, 38) des Schaftes (18) eine hochpolierte Oberfläche aufweisen.

**Claims**

1. A femoral hip-joint prosthesis (10) for implantation by cement, having a collarless shoulder (34) at the proximal end and having a stem (18) which extends in a straight line from the shoulder (34) to the distal end (46) and tapers from the proximal end toward the distal end (46),characterized in that the stem (18) has a quadrilateral cross section (40) and an anterior side face (28) and a posterior side face (28'), that the two side faces (28, 28'), in the direction of extension of the stem (18), have, in a first subsidiary section (26) beginning at the shoulder (34), a logarithmically extending curvature, and that the two side faces (28, 28') thereafter, in a second subsidiary section (32), extend in a straight line and converge.

2. The femoral hip-joint prosthesis (10) as claimed in claim 1, characterized in that the stem (18) has medial and lateral side faces (36, 38), the side faces (36, 38) each having, in the direction of extension of the stem (18), in a first subsidiary section (26) beginning at the shoulder, a logarithmically extending curvature, and thereafter, in a second subsidiary section (32), extending in a straight line and converging.

3. The femoral hip-joint prosthesis (10) as claimed in one of the preceding claims, characterized in that the second subsidiary section (32), starting from the distal end (46), extends along more than half, preferably a third, of the stem (18).

4. The femoral hip-joint prosthesis (10) as claimed in one of the preceding claims, characterized in that the quadrilateral cross section (40) forms edges (44) which are rounded.

5. The femoral hip-joint prosthesis (10) as claimed in claim 4, characterized in that the rounded edges (44) have a radius of between 1 and 3 mm.

6. The femoral hip-joint prosthesis (10) as claimed in one of the preceding claims, characterized in that the quadrilateral cross section (40) is trapezoidal, rectangular or square, the cross section (40), from the proximal end to the distal end (46) of the stem (18), being able to merge from one into another of these shapes.

7. The femoral hip-joint prosthesis (10) as claimed in one of the preceding claims, characterized in that the stem (18) has a third, distal end section with a circular cross section (40).

8. The femoral hip-joint prosthesis (10) as claimed in one of the preceding claims, characterized in that the side faces (28, 28' and 36, 38), starting from the distal end (46), extend parallel to one another along a length of up to 5 mm.

9. The femoral hip-joint prosthesis (10) as claimed in one of the preceding claims, characterized in that at least the side faces (28, 28', 36, 38) of the stem (18) have a highly polished surface.

## Revendications

1. Prothèse fémorale de l'articulation de la hanche (10) pour l'implantation au moyen de ciment, présentant un épaulement sans collet (34) au niveau de l'extrémité proximale et une tige (18) s'étendant en ligne droite de l'épaulement (34) vers l'extrémité distale (46), qui se rétrécit de l'extrémité proximale vers l'extrémité distale (46), caractérisée en ce que la tige (18) présente une section transversale (40) quadrangulaire ainsi qu'un flanc antérieur (28) et un flanc postérieur (28'), en ce que les deux flancs (28, 28') dans la direction de l'extension de la tige (18) présentent, dans une première section partielle (26) commençant au niveau de l'épaulement (34) une courbure d'allure logarithmique, et en ce que les deux flancs (28, 28') s'étendent ensuite dans une deuxième section partielle (32) en ligne droite et en convergeant.

2. Prothèse fémorale de l'articulation de la hanche (10) selon la revendication 1, caractérisée en ce que la tige (18) présente un flanc médian (36) et un flanc latéral (38), les flancs (36, 38) présentant chacun dans la direction de l'extension de la tige (18), dans une première section partielle (26) commençant au niveau de l'épaulement, une courbure d'allure logarithmique, et ensuite, dans une deuxième section partielle (32), s'étendent en ligne droite et en convergeant.

3. Prothèse fémorale de l'articulation de la hanche (10) selon l'une quelconque des revendications précédentes, caractérisée en ce que la deuxième section partielle (32), partant de l'extrémité distale (46), s'étend sur la moitié, de préférence sur un tiers de la tige (18).

4. Prothèse fémorale de l'articulation de la hanche (10) selon l'une quelconque des revendications précédentes, caractérisée en ce que la section transversale quadrangulaire (40) forme des arêtes (44) qui sont arrondies.

5. Prothèse fémorale de l'articulation de la hanche (10) selon la revendication 4, caractérisée en ce que les arêtes arrondies (44) présentent un rayon entre 1 et 3 mm.

6. Prothèse fémorale de l'articulation de la hanche (10) selon l'une quelconque des revendications précédentes, caractérisée en ce que la section transversale quadrangulaire (40) est trapézoïdale, rectangulaire ou carrée, la section transversale (40) de l'extrémité proximale à l'extrémité distale (46) de la tige (18) pouvant passer de l'une de ces formes à une autre.

7. Prothèse fémorale de l'articulation de la hanche (10) selon l'une quelconque des revendications précédentes, caractérisée en ce que la tige (18) présente une troisième section d'extrémité distale avec une section transversale (40) de configuration circulaire.

8. Prothèse fémorale de l'articulation de la hanche (10) selon l'une quelconque des revendications précédentes, caractérisée en ce que les flancs (28, 28' et 36, 38) s'étendent depuis l'extrémité distale (46) sur une longueur pouvant aller jusqu'à 5 mm parallèlement l'un à l'autre.

9. Prothèse fémorale de l'articulation de la hanche (10) selon l'une quelconque des revendications précédentes, caractérisée en ce qu'au moins les flancs (28, 28', 36, 38) de la tige (18) présentent une surface très polie.

Fig.3

Fig.2a (A-A)

Fig.2b (B-B)

Fig.1

8